# EUROPEAN PATENT APPLICATION

(11) **EP 4 670 776 A1**
(43) Date of publication of application: **31.12.2025**
(21) Application number: 24796097.4
(22) Date of filing: 24.04.2024
(51) Int. Cl.: A61M 60/178

(54) **CATHETER PUMP**

(30) Priority: 26.04.2023 CN 202310459725; 26.04.2023 CN 202310459666
(71) Applicant: Magassist Co., Ltd., Suzhou, Jiangsu 215163 (CN)
(72) Inventor: TU, Pan, Suzhou, Jiangsu 215163 (CN); YEN, Ifan, Suzhou, Jiangsu 215163 (CN)
(74) Representative: Grünecker Patent- und Rechtsanwälte PartG mbB
(86) International application number: PCT/CN2024/089475
(87) International publication number: WO 2024/222718

(57) **Abstract**

A catheter pump, comprising: a catheter, and a pump head that can be delivered to a desired position of the heart by means of the catheter so as to pump blood. The pump head comprises a pump housing having a blood inlet and a blood outlet and an impeller housed in the pump housing. The impeller is driven to rotate so as to suck blood via the blood inlet into the pump housing and then pump out the blood via the blood outlet. The pump housing comprises a stent, the stent being operably switched between a radially retracted state and a radially deployed state. In the radially deployed state, the stent comprises a substantially cylindrical body portion, an inlet portion located at the distal end in the axial direction of the body portion, and an outlet portion located at the proximal end in the axial direction of the body portion, the inlet portion and the outlet portion being connected and supporting the body portion. The body portion is provided with a plurality of meshes, each mesh being defined by at least two pairs of oppositely-arranged side edges. All the side edges have the same radial thickness, and the circumferential width of the two ends in the axial direction of each side edge is larger than the circumferential width of the middle of same.

## Description

This application claims the priorities to the following two Chinese patent applications, both of which are incorporated herein by reference in their entirety:
Chinese Patent Application No. 202310459666.2, titled "CATHETER PUMP", filed with the China National Intellectual Property Administration on April 26, 2023; and
Chinese Patent Application No. 202310459725.6, titled "CATHETER PUMP", filed with the China National Intellectual Property Administration on April 26, 2023.

### FIELD

The present application relates to the technical field of medical devices, and in particular to a catheter pump.

### BACKGROUND

Catheter pumps are classified into non-collapsible and collapsible types. The collapsible catheter pump, causing smaller incisions during intervention, is more convenient and quick to use.

A stent is a critical component in realizing the collapsibility of the catheter pump. The stent typically includes a pump portion located at its middle part and having a substantially cylindrical shape, and inlet and outlet portions located at both ends of the pump portion in an axial direction and having substantially conical shapes. An impeller is mainly located in the pump portion. The inlet and outlet portions include bars which are connected to and support the pump portion to maintain its shape.

The conventional stent is basically designed to have bars with a uniform width in the pump portion. Specifically, during the collapse or expansion of the stent, every two adjacent bars in the pump portion are turned close to each other (in a case of collapse) or away from each other (in a case of expansion) at connection points of their ends. Such turning occurring at the ends of the bars may cause stress fatigue at the ends, which in turn leads to a decrease in the rigidity at the ends of the bars.

With the same material and radial thickness, the bars with the uniform width in the pump portion of the stent mean that the bars in the pump portion each has the same overall rigidity. In this case, the aforementioned turning at the ends may result in a decrease in the rigidity at the ends due to the stress fatigue, while the middle portion, which only undergoes passive turning, is essentially unaffected in terms of rigidity. Consequently, after multiple collapses and expansions of the stent, the rigidity at the ends of the bars decreases, and other components connected to the ends, such as adjacent edge struts and axial connecting bars, may break, resulting in structural instability of the stent and shortening service life of the stent.

### SUMMARY

**In** view of deficiencies in the conventional technology, an object of the present application is to provide a catheter pump capable of compensating for the rigidity at ends of edge struts, thereby guaranteeing the structural stability of a stent, and prolonging the service life of the stent.

A catheter pump includes a catheter, and a pump head configured to be delivered through the catheter to a desired location in a heart for pumping blood. The pump head includes a pump housing having at least one blood inlet and at least one blood outlet, and an impeller housed in the pump housing. The impeller is driven to rotate to draw blood through the blood inlet into the pump housing and discharge blood through the blood outlet.

The pump housing includes a stent operable between a radially collapsed state and a radially expanded state. The stent in the radially expanded state includes a body portion having a substantially cylindrical shape, an inlet portion located at a distal end of the body portion in an axial direction, and an outlet portion located at a proximal end of the body portion in the axial direction. The inlet and outlet portions are connected to and support the body portion. The body portion is provided with multiple meshes, each defined by at least two pairs of oppositely-arranged edge struts. All of the edge struts have a same radial thickness. Each of the edge struts has a circumferential width (a width in a circumferential direction) greater at both ends than at a middle portion in an axial direction of the edge strut.

In an example, the circumferential width of the edge strut decreases from the both ends to the middle portion in a curved manner.

In an example, the edge strut has two circumferentially opposite sides that gradually narrow from the both ends to the middle portion.

In an example, the two circumferentially opposite sides of the edge strut narrow from the both ends to the middle portion at a same tendency and to a same extent.

In an example, the edge strut has the narrowest circumferential width approximately at its middle portion.

In an example, the circumferential width of the edge strut close to a middle part of the body portion is greater than that of the edge strut away from the middle part of the body portion.

In an example, the edge struts located at a same position in an axial direction of the body portion have a same circumferential width.

In an example, a minimum circumferential width of the edge strut close to a middle part of the body portion is greater than or equal to a maximum circumferential width of the edge strut away from the middle part of the body portion.

In an example, multiple edge struts located at a same position in the axial direction are connected end to end in a circumferential direction to form a serrated ring, and multiple serrated rings are arranged and connected together in the axial direction. The serrated ring has multiple distal apexes and multiple proximal apexes arranged alternately in the circumferential direction. Multiple axial connecting bars are provided between every two adjacent serrated rings and are arranged in the circumferential direction. Opposite ends of the axial connecting bar are respectively connected to the distal apex and the proximal apex opposite to each other in the axial direction. A circumferential width of the axial connecting bar close to a middle part of the body portion is greater than that of the axial connecting bar away from the middle part of the body portion.

In an example, on a same serrated ring, ends of every two adjacent edge struts are spaced apart in the circumferential direction.

In an example, a first avoidance slot is formed at ends of the every two adjacent edge struts between which an angle is formed, and extends in the axial direction. The first avoidance slot has opposite sides that are substantially parallel over a portion of its length.

With the catheter pump according to the present embodiment, each of the edge struts has a circumferential width greater at the both ends than at the middle portion in the axial direction of the edge strut. That is, for any edge strut, the circumferential width of the edge strut gradually decreases from the both ends to the middle portion. Alternatively, the circumferential width of the edge strut at the middle portion is smaller than that at the both ends. The reduced rigidity at the ends due to the stress fatigue can be compensated by increasing the width of the edge strut at the ends where it is most vulnerable. In this way, after multiple collapses and expansions of the stent, the ends of the edge strut maintains high rigidity, preventing the ends of the edge strut from breaking from other connected components, such as the adjacent edge struts and the axial connecting bars, thereby guaranteeing the structural stability of the stent and prolonging the life of the stent.

With the radial thickness of the edge strut remaining unchanged, the gradually decreasing of the width at a middle region weakens the structural strength of the edge strut in the middle region, so that stress concentration is more likely to occur in the middle region than at the ends. Therefore, the stress and strain generated at the ends of the edge strut can be directed to the middle region, so that the stress is no longer concentrated solely at the ends of the edge strut, but is partially directed or transferred to the middle region. Consequently, the stress distribution across the overall structure of the edge strut tends to be uniform. That is, by using an improved stent with the edge strut having a decreased width at the middle portion, the stress that would otherwise be concentrated at the connection point (proximal or distal apex) is directed to the middle region of the edge strut during the collapse or expansion of the stent, making the stress distribution across the edge strut more uniform, thereby prolonging the life of the stent.

### BRIEF DESCRIPTION OF THE DRAWINGS

FIG. 1 is a schematic structural view showing a stent according to an embodiment of the present application;
FIG. 2 is an enlarged front view showing a part of the stent in FIG. 1;
FIG. 3 is a partially enlarged view of FIG. 2;
FIG. 4a is a schematic structural view showing a catheter pump according to an embodiment of the present application;
FIG. 4b is a schematic structural view showing a catheter pump according to another embodiment of the present application;
FIG. 5 is a partial cross-sectional view of FIG. 4b;
FIG. 6 is a schematic view showing a stent in the conventional technology exhibiting a "Dogbone" phenomenon when subjected to radial external forces;
FIG. 7 is a schematic view showing self-interference of the stent in the conventional technology when collapsed;
FIG. 8 is a diagram illustrating stress hotspots of edge struts of a standard stent during collapse or expansion;
FIG. 9 is a diagram illustrating stress hotspots of edge struts of a stent according to an embodiment of the present application during collapse or expansion;
FIG. 10 is a stress-strain diagram of the edge struts of the standard stent during the collapse or expansion;
FIG. 11 is a stress-strain diagram of the edge struts of the stent according to an embodiment of the present application during the collapse or expansion;
FIG. 12 is a schematic structural view showing a stent according to another embodiment of the present application; and
FIGS. 13A to 13C are schematic diagrams showing three different connections of the edge struts to avoid self- interference.

Reference numerals in the drawings are as follows:

| | | | |
|---|---|---|---|
| 1000 | catheter pump; | 100 | power assembly; |
| 101 | housing; | 200 | working assembly; |
| 201 | catheter; | 202 | driving shaft; |
| 2021 | flexible shaft; | 2022 | rigid shaft; |
| 204 | driving catheter handle; | 205 | pump head; |
| 2051 | pump housing; | 2051a | blood inlet; |
| 2051b | blood outlet; | 20511 | stent; |
| 20512 | film; | 2052 | impeller; |
| 20521 | hub; | 20522 | blade; |
| 206 | proximal bearing housing; | 207 | distal bearing housing; |
| 208 | proximal bearing; | 209 | distal bearing; |
| 210 | non-invasive support member; | 211 | stopper; |
| 212 | position-limiter; | 11 | body portion; |
| 12 | inlet portion; | 13 | outlet portion; |
| 14 | mesh; | 111 | edge strut; |
| 15 | first edge strut; | 16 | second edge strut; |
| 17 | serrated ring; | 18 | distal apex; |
| 19 | proximal apex; | 21 | axial connecting bar; |
| 271 | first avoidance slot; | 272 | first strut; |
| 273 | first intersection; | 274 | second avoidance slot; |
| 275 | second strut; | 276 | second intersection; |
| 277 | third avoidance slot; | 22 | distal connecting portion; |
| 221 | distal connecting leg; | 222 | distal bar body; |
| 223 | third bar portion; | 224 | fourth bar portion; |
| 225 | distal transition portion; | 226 | second extension; |
| 23 | proximal connecting portion; | 231 | proximal connecting leg; |
| 232 | proximal bar body; | 233 | first bar portion; |
| 234 | second bar portion; | 235 | proximal transition portion; |
| 236 | first extension; | X | axial direction. |

### DETAILED DESCRIPTION OF THE EMBODIMENTS

The present application is described in detail hereinafter with reference to embodiments shown in the drawings. However, these embodiments do not limit the present application, and any changes to structure, method, or function made by those skilled in the art based on these embodiments are all within the protection scope of the present application.

The terms such as "proximal", "distal", "front", and "rear" used in the present application are defined relative to a clinician operating a catheter pump 1000 in the embodiments. The terms "proximal" and "rear" refers to a portion relatively close to the clinician, and the terms "distal" and "front" refers to a portion relatively away from the clinician. For example, a portion outside the body is at a proximal or rear end, and a portion inserted into the body is at a distal or front end.

It should be understood that orientations such as "proximal", "distal", "rear", and "front" are defined for convenience of description. However, the catheter pump 1000 may be used in various directions and positions, so these terms expressing relative positional relationships are not restrictive or absolute. For example, these definitions to various directions are merely for the convenience of illustrating technical solutions of the present application and do not limit the direction of the catheter pump 1000 in other scenarios, including but not limited to product testing, transportation, and manufacturing, which may cause the catheter pump 1000 to be inverted or repositioned. In the present application, these definitions shall comply with above explicit provisions or limitations if they are otherwise explicitly specified or limited.

In the present application, terms such as "linkage" and "connection" should be interpreted broadly, unless otherwise explicitly specified or limited. For example, it may refer to a fixed connection, a detachable connection, a movable connection, or connection as an integral; may refer to a direct connection or an indirect connection through an intermediate medium; and may refer to internal communication between two components or interaction between two components. Those skilled in the art can understand meanings of these terms in the present application based on specific conditions.

In addition, technical features involved in the different embodiments of the present application described below may be combined with each other as long as they do not conflict with each other.

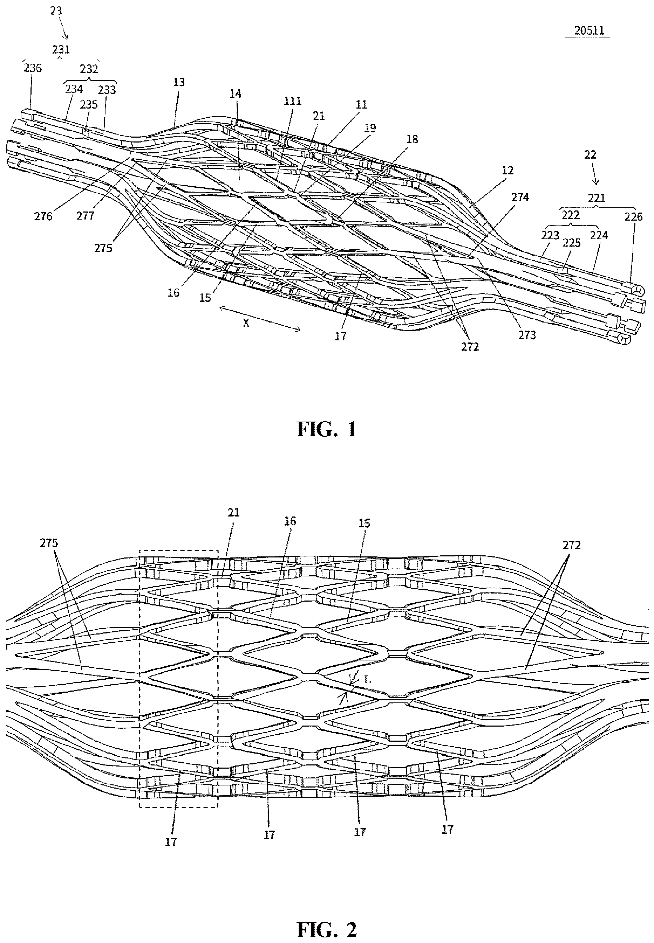

Referring to FIGS. 1 to 3, a stent 20511 according to an embodiment of the present application is operable between a radially collapsed state and a radially expanded state. The stent 20511 in the radially expanded state includes a body portion 11 having a substantially cylindrical shape, and an inlet portion 12 and an outlet portion 13 respectively located at distal and proximal ends of the body portion 11 in an axial direction X and having substantially conical shapes. The inlet portion 12 and the outlet portion 13 are connected to and support the body portion 11.

The body portion 11 is provided with multiple meshes 14, each defined by at least two pairs of oppositely-arranged edge struts 111. Multiple edge struts 111 located at a same position in the axial direction X are sequentially connected end to end in a circumferential direction to form a serrated ring 17. Multiple serrated rings 17 are arranged and connected together in the axial direction X. The edge strut 111 of the serrated ring 17 at a middle part has a maximum circumferential width (a width in a circumferential direction) greater than that of the edge strut 111 of the serrated ring 17 away from the middle part. The maximum circumferential width of the edge strut 111 may be simply understood as a circumferential width of the edge strut 111.

As shown in FIG. 2, the serrated ring 17 is formed by sequentially connecting first edge struts 15 and second edge struts 16, which are located at the same position in the axial direction, end to end in the circumferential direction. The first edge struts 15 and the second edge struts 16 are connected to each other at an angle, thereby forming a circumferentially continuous ring that is tooth-shaped or partially W-shaped (as shown in a dotted box in FIG. 2).

The circumferential width of the edge strut 111 refers to a dimension of the edge strut 111 in the circumferential direction, such as a width L indicated by an arrow in FIG. 2. Certainly, the circumferential width of other parts, such as connecting legs and components included therein, and axial connecting bars, of the stent 20511 can be understood similarly.

The circumferential width of the edge strut 111 refers to a width dimension of the edge strut 111 in the circumferential direction of the stent 20511. The middle part refers to a middle part of the body portion 11 in the axial direction X, specifically a regional position where any serrated rings 17/edge struts 111, excluding those located at both ends (proximal and distal ends) of the body portion 11, are located. Accordingly, the serrated rings 17/edge struts 111 located at the middle part refer to any serrated rings 17/edge struts 111 in a region excluding any serrated rings 17/edge struts 111 located at the both ends (proximal and distal ends).

For example, in an embodiment as shown in FIGS. 1 and 2 with four serrated rings 17, the middle part is a regional position where two intermediate serrated rings 17 are located. Alternatively, the four serrated rings 17 may be numbered sequentially in the axial direction (either from proximal to distal or from distal to proximal), with the serrated rings 17 numbered 1 and 4 located at end positions and the serrated rings 17 numbered 2 and 3 located at the middle part.

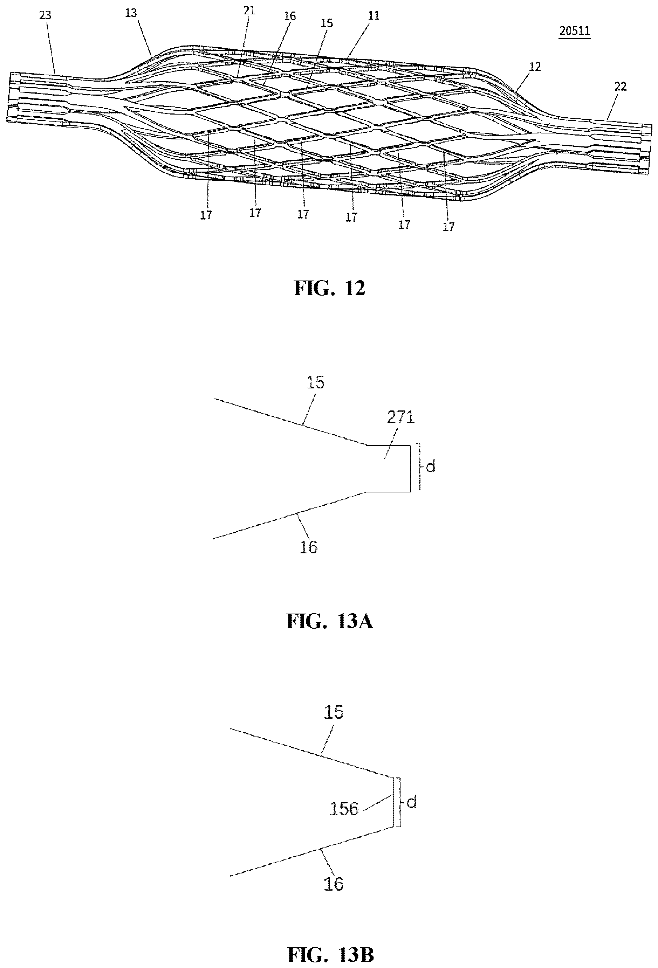

Similarly, in an embodiment as shown in FIG. 12 with six serrated rings 17, the middle part is a regional position where four intermediate serrated rings 17 are located. Alternatively, the six serrated rings 17 are numbered sequentially, with the serrated rings 17 numbered 1 and 6 located at the end positions, and the serrated rings 17 numbered 2, 3, 4, and 5 located at the middle part.

That is, in the axial direction X, the circumferential width of the edge strut 111 located at the both ends of the body portion 11 is smaller than that of the edge strut 111 located between the both ends of the body portion 11. The circumferential width of the edge strut 111 increases towards the middle part of the body portion 11, and decreases towards the proximal end or edge positions of the body portion 11. Thus, when all of the edge struts 111 of the body portion 11 have the same radial thickness, the edge struts 111 at the both ends of the body portion 11 of the stent 20511 have relatively weaker rigidity, and the edge struts 111 at the middle part have relatively stronger rigidity. In this way, the body portion 11 has a smaller rigidity at the both ends, facilitating the collapse of the stent 20511.

Research has revealed that a greatest resistance is generated at the both ends of the body portion 11 when the stent 20511 is collapsed, rather than at the middle part of the body portion 11. Thus, with the stent 20511 according to the present embodiment, the maximum circumferential width of the edge strut 111 located at the middle part of the body portion 11 is provided to be greater than that of the edge strut 111 located at the both ends of the body portion 11, that is, the circumferential width of the edge strut 111 located at the middle part of the body portion 11 of the stent 20511 is greater than that of the edge strut 111 located at the both ends of the body portion 11, which effectively reduces the resistance to collapse while guaranteeing the rigidity of the stent 20511. Therefore, the stent 20511 provided according to the present embodiment has sufficient rigidity and facilitates easy collapse.

In the present embodiment, the "rigidity" specifically refers to the ability of the stent 20511 to resist deformation due to a radial external force when in the radially expanded state (especially in an operating state). With higher rigidity, the stent 20511 has better resistance to deformation under the radial external force, or experiences less radial inward deformation under the same radial external force. Conversely, with lower rigidity, the stent 20511 has less resistance to deformation under the radial external force, or experiences more radial inward deformation under the same radial external force.

Specifically, as described below, when a pump head 205 of the catheter pump 1000 of the stent 20511 according to the present embodiment is introduced into the ventricle and the impeller 2052 rotates to pump blood, the pump head 205 may swing in the ventricle due to certain reasons, such as patient movement or cardiac function, potentially causing lateral impact on the ventricular wall. If the stent 20511 lacks sufficient rigidity, such lateral impact may cause the stent 20511 to radially concave, leading to the rotating impeller 2052 scraping against the stent 205 11, which is undesirable because the impeller 2052 may be entangled with the stent 20511, forcing the impeller 2052 to stop and fail to pump blood. According to the present embodiment, the edge strut 111, at the middle part of the body portion 11, has greater circumferential width, so that the body portion 11 has greater rigidity in a middle region for primarily housing the impeller 2052, which provides the stent 20511 with superior expansion rigidity, and significantly mitigates the issues that the pump head 205 radially concaves when subjected to the lateral impact, thereby avoiding the impeller 2052 from contacting an inner wall of the stent 20511 and preventing the impeller 2052 from being forced to stop and fail to pump blood.

Since the body portion 11 of the stent 20511 is connected to the inlet portion 12 and the outlet portion 13 at the both ends in the axial direction X, and generally, bars included in the inlet portion 12 and the outlet portion 13 have a relatively large circumferential width, the meshes or bars at the both ends of the body portion 11 in the axial direction may be supported by the inlet portion 12 and the outlet portion 13 which have a relatively large rigidity. Consequently, even though the edge struts 111 located at the both ends have small circumferential width, the rigidity of the stent 20511 is not significantly compromised. With the body portion 11 supported at the both ends by the inlet portion 12 and the outlet portion 13, the rigidity at the both ends of the body portion 11 is positively compensated to a certain extent. As a result, the overall rigidity of the body portion 11 tends to be uniform, maximally preventing a "Dogbone" phenomenon from occurring when the stent 20511 is collapsed or subjected to a lateral force.

Furthermore, since the stent 20511 is collapsed from the ends, the high rigidity at the ends of the body portion 11 in the axial direction actually hinders the collapse of the stent 20511. Therefore, the above solution appropriately reduces the rigidity at the ends of the body portion 11 in the axial direction, facilitating the collapse of the stent 20511.

As described above, considering only the body portion 11, since the meshes or bars at its both ends in the axial direction are supported by the inlet portion 12 and the outlet portion 13 which have higher rigidity, the body portion 11 exhibits higher rigidity at the both ends, while the meshes or bars at a middle part may only be supported by the meshes or bars at the both ends. Therefore, if the structure and material of the body portion 11 are uniform, the rigidity at the both ends of the body portion 11 is greater than that at the middle part.

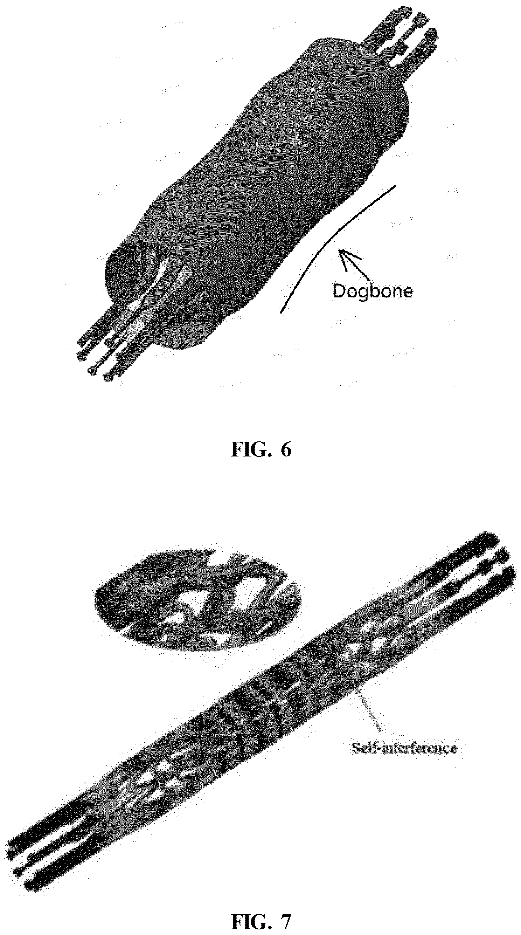

Practice has shown that if the body portion 11 is designed to have the edge struts with the same circumferential width at the both ends and the middle part, the body portion 11 would exhibit a "Dogbone" phenomenon when the pump head 205 is subjected to the radial external force, with the middle part becoming concave and the both ends remaining essentially unchanged, as shown in FIG. 6. Since the impeller 2052 is mostly located in the middle region of the body portion 11, the "Dogbone" phenomenon easily leads to the aforementioned issue of the impeller 2052 being entangled with the stent 20511.

With the above-mentioned structural design of the present embodiment, the maximum circumferential width of the edge strut 111 of the serrated ring 17 located at the middle part is greater than that of the edge strut 111 of the serrated ring 17 located at the both ends, that is, the rigidity of the edge strut 111 at the both ends of the body portion 11 is appropriately decreased, and the rigidity of the edge strut 111 at the middle part is appropriately increased, so that the overall rigidity of the body portion 11 is uniform, avoiding the "Dogbone" phenomenon and facilitating the collapse of the stent 20511 and providing the stent 20511 with superior expansion rigidity.

In addition, the minimum circumferential width of the edge strut 111 at the end positions is smaller than that of the edge strut 111 at the middle part. In this way, in the same comparison dimension, the circumferential width of the edge strut 111 at the end positions is smaller than that of the edge strut 111 at the middle part, thereby guaranteeing uniform support rigidity of the body portion 11.

The circumferential width of the edge strut 111 may be uniform, in which case, the "minimum circumferential width" and the "minimum circumferential width" of the edge strut described above are equal and are a fixed constant value. Certainly, the circumferential width of the edge strut 111 may vary (as described below). In this case, comparing the edge struts 111 at different positions in terms of either maximum circumferential width or minimum circumferential width, it ensures that the edge struts 111 at the middle part and the edge struts 111 at the end positions are compared at the same dimension, thereby ensuring that the comparison in terms of the circumferential width is meaningful

In the present embodiment, all of the edge struts 111 included in the same serrated ring 17 have the same circumferential width at the same position in the axial direction X, so that the rigidity of the stent 20511 is uniform at the same position in the axial direction X.

In the present embodiment, each of the edge struts 111 has a circumferential width that is greater at its both ends than at its middle portion in the axial direction. That is, for an edge strut 111, the circumferential width of the edge strut 111 gradually decreases from the both ends to the middle portion. Alternatively, the circumferential width of the edge strut 111 is smaller at the middle portion than at the both ends. The gradually decreasing of the width of the edge strut 111 from the both ends to the middle portion specifically includes decreasing in a linear manner or in a curved manner.

Practice has shown that the above structural design makes the rigidity of each of the edge struts 111 tend to be consistent, thereby improving the structural stability of the stent 20511 (specifically, preventing the stent 20511 from becoming loose or disconnected) and prolonging the life of the stent 20511.

Specifically, both ends of the edge strut 111 serve as connection points (connected to an axial connecting bar 21 and to an adjacent edge strut 111). During the collapse or expansion of the stent 20511, every two adjacent edge struts 111 turn close to each other (corresponding to the collapsed state) or away from each other (corresponding to the expanded state) at the connection points of the ends. Such turning occurring at the ends may cause stress fatigue at the ends of the edge strut 111, thereby decreasing the rigidity of the ends of the edge strut 111.

When the radial thickness is constant, the overall rigidity of the edge strut 111 is consistent if the edge strut 111 has a uniform width (hereinafter referred to as a standard stent). In this case, the aforementioned turning of the ends of the edge strut 111 would decrease the rigidity of the ends due to the stress fatigue, while the middle portion, which only undergoes passive turning, is essentially unaffected in terms of rigidity.

Therefore, with the aforementioned solution, the width of the edge strut 111 is increased at the ends where it is most vulnerable (hereinafter referred to as an improved stent), to compensate for the reduced rigidity at the ends due to the stress fatigue. In this way, after multiple collapses and expansions of the stent 205 11, the ends of the edge strut 111 maintains high rigidity, preventing the ends of the edge strut 111 from breaking from other connected components, such as the adjacent edge strut 111 and the axial connecting bar 21, thereby guaranteeing the structural stability of the stent 20511 and prolonging the life of the stent 20511.

In addition, by using the improved stent with a smaller width at the middle portion of the edge strut 111, the stress that would otherwise be concentrated at the connection point (the proximal apex or the distal apex) is directed to the middle portion of the edge strut 111 during the collapse or expansion of the stent 205 11, making the stress distribution across the edge strut 111 more uniform, thereby prolonging the life of the stent 20511.

Specifically, with two circumferentially opposite sides of the edge strut 111 gradually narrowing from the both ends towards the middle portion, rather than only one of the two sides narrowing, the rigidity of each of the edge struts, when viewed form the same position in the axial direction, is uniformly distributed in the circumferential direction, the two sides exhibit equivalent rigidity and uniform stress distribution at corresponding positions, and the rigidity at the ends of the two sides is compensated, thereby guaranteeing the structural stability of the stent 20511 and prolonging the service life of the stent 20511.

Furthermore, the two circumferentially opposite sides of the edge strut 111 are symmetrically arranged, that is, the two circumferentially opposite sides of the edge strut 111 narrow from the both ends to the middle portion at a same tendency and to a same extent, thereby allowing the two sides to have the same rigidity at the corresponding positions in an extension direction of the edge strut 111. Specifically, the two circumferentially opposite sides of the edge strut 111 are symmetrically arranged with respect to a central plane, which is parallel to the extension direction and the radial thickness direction of the edge strut 111 and passes through a center of the edge strut 111.

As shown in FIG. 2, the edge strut 111 has the narrowest circumferential width at approximately the middle portion of the edge strut 111 itself. A part of the edge strut 111 with the narrowest circumferential width may be a specific point or a region. When the part of the edge strut 111 with the narrowest circumferential width is a point, the point is preferably the center in the extension direction of the edge strut 111. If the part of the edge strut 111 with the narrowest circumferential width is a region, the region may be around the center in the extension direction of the edge strut 111, and the edge strut 111 has the same circumferential width in the region, which is a minimum circumferential width of the edge strut 111. Preferably, the center of the region is the center in the extension direction of the edge strut 111.

In the present embodiment, the minimum circumferential width of the edge strut 111 close to the middle part of the body portion 11 is greater than or equal to the maximum circumferential width of the edge strut 111 away from the middle part. In this way, considering only the body portion 11 (that is, without considering the support for the body portion 11 provided by the inlet portion 12 and the outlet portion 13), the rigidity of the body portion 11 is smaller at the both ends than at the middle part. Therefore, combined with the support provided by the inlet portion 12 and the outlet portion 13 to the both ends of the body portion 11, the overall rigidity of the body portion 11 is tended to be consistent, thereby achieving the effects described above.

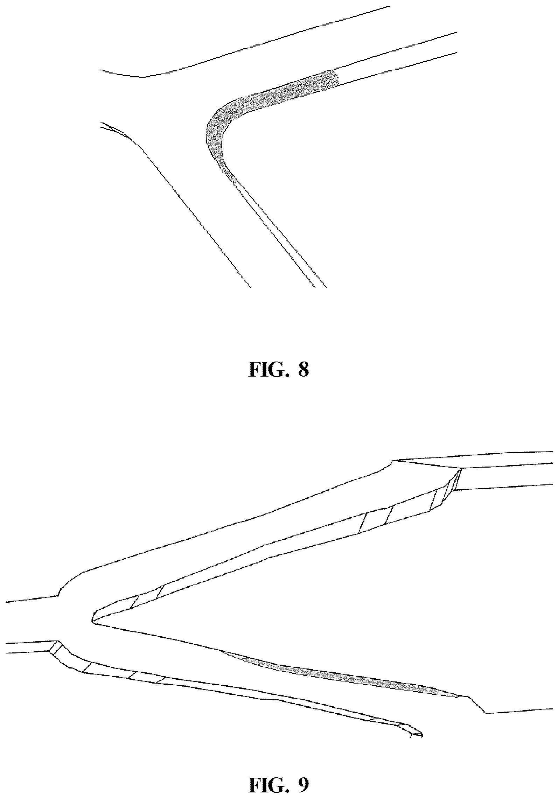

FIGS. 8 and 9 are diagrams illustrating the approximate distribution of stress hotspots of the edge struts of the standard stent and the improved stent during the collapse and expansion, as concluded by the applicant through experimental simulations. As shown in FIG. 8, the stress hotspots of the standard stent are concentrated in an apex region, with the middle region experiencing little strain. In contrast, the stress hotspots of the improved stent are successfully directed to the middle region, resulting in a more even stress distribution. Furthermore, a maximum stress in the apex region has been significantly reduced compared with the standard stent.

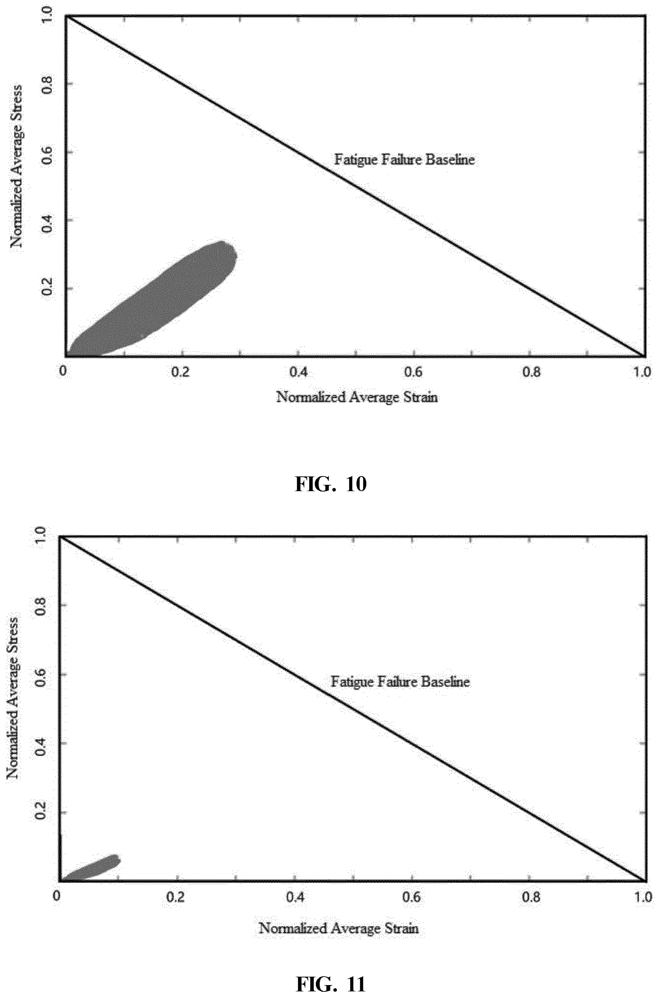

In addition, FIGS. 10 and 11 are stress-strain diagrams of the edge strut 111 of the standard stent and the improved stent during the collapse and expansion, as concluded by the applicant through testing and verification. As can be seen, under identical test conditions (including the same number of collapses and expansions, identical radial thickness and identical end thickness for the standard and improved stents), the maximum stress of the edge strut 111 of both the standard and improved stents does not reach or exceed a fatigue failure baseline. This indicates that both stents are able to withstand cyclical loads without failure under the specified testing conditions.

Compared with the standard stent, however, a maximum stress-strain scatter point of the improved stent is significantly below the fatigue failure baseline. Furthermore, both the stress and strain of the standard stent excessively exceed those of the improved stent, and the upward slope of its test scatter points is steeper than that of the improved stent. This suggests that the improved stent would undergo significantly more collapses and expansions than the standard stent to cause the stress of the edge strut to reach the fatigue failure baseline and cause the edge strut to fracture. Therefore, the fatigue resistance of the improved stent is significantly superior to that of the standard stent, which is beneficial for prolonging the service life of the stent and enhancing safety.

Through multiple designs and verifications, the inventors of the present application have found that the aforementioned improved stent achieves a surprisingly uniform stress distribution on the edge strut 111. The inventors have been investigating the underlying principle behind this surprising effect, but have yet to reach a definitive conclusion. The inventors suggest that the underlying principle may be as follows: when a component exhibits non-uniform structural strength, stress concentration tends to occur in locally weaker regions. Specifically in the present embodiment, with the radial thickness of the edge strut 111 remains unchanged, the gradually narrowing of the width in the middle region weakens the structural strength of the edge strut 111 in the middle region, so that stress concentration is more likely to occur in the middle region than at the ends. Therefore, the stress and strain generated at the ends of the edge strut 111 can be directed to the middle region, so that the stress is no longer concentrated solely at the ends of the edge strut 111, but is partially directed or transferred to the middle region. Consequently, the stress distribution across the overall structure of the edge strut 111 tends to be uniform.

In the present embodiment, the narrowing of the edge strut 111 may be linear, preferably curvilinear. While the linear narrowing creates an angle induced by a sudden change in shape in the middle portion of the edge strut 111, the curvilinear narrowing allows for smoother change in the circumferential sides of the edge strut 111, eliminating the aforementioned sudden change in shape. Consequently, stress distribution is more uniform. Practice has also shown that, compared with the linear narrowing, the curvilinear narrowing of the edge strut 111 exhibits a higher safety factor and a lower probability of fracture.

The minimum circumferential width of the edge strut 111 at the middle part of the body portion 11 is greater than or equal to the maximum circumferential width of the edge strut 111 at the ends of body portion 11. In this way, considering only the body portion 11 (that is, without considering the support for the body portion 11 provided by the inlet portion 12 and the outlet portion 13), the rigidity of the body portion 11 is smaller at the both ends than at the middle part. Therefore, combined with the support provided by the inlet portion 12 and the outlet portion 13 to the both ends of body portion 11, the overall rigidity of the body portion 11 is tended to be consistent.

As described above, when the circumferential width of the edge strut 111 is uniform, the minimum and maximum circumferential widths of the edge strut 111 at the middle part are the same constant, denoted as C1. Similarly, the maximum and minimum circumferential widths of the edge strut 111 at the ends are the same constant, denoted as C2, where C1 is greater than C2.

When the circumferential width of the edge strut 111 varies, the minimum circumferential width of the edge strut 111 at the middle part is presented in the middlemost of the edge strut 111, and the maximum circumferential width of the edge strut 111 at the end positions is presented at the both ends of the edge strut 111. Specifically, the circumferential width of the edge strut 111 at the middle part gradually decreases from a maximum value C3 at the ends to a minimum value C4 in the middlemost position. The circumferential width of the edge strut 111 at the ends gradually decreases from a maximum value C5 at the ends to a minimum value C6 at the middlemost position. C4 is greater than or equal to C5. The relationship among C3 to C6 is : C3 > C4 ≥ C5 > C6.

It should be noted that all of the edge struts 111 of the body portion 11 have the same radial thickness. In this way, by adjusting only the circumferential width of the edge strut 111, the rigidity of the stent 20511 can be adjusted in respective positions, thereby making the rigidity adjustment of the body portion 11 simple and flexible.

Furthermore, all of the axial connecting bars 21 of the body portion 11 also have the same radial thickness, which is identical to the radial thickness of the edge struts 111, so as to match the edge struts 111 for the width adjustment at different positions in the axial direction, thereby achieving the rigidity adjustment of the body portion 11.

Furthermore, the radial thickness of the edge strut 111 is equal to that of solid structures (first struts 272 described below) defining inlet meshes of the inlet portion 12, and that of solid structures (second struts 275 described below) defining outlet meshes of the outlet portion 13. In other words, the solid structures of the stent 20511 have the same and uniform thickness at all positions in the axial direction. Consequently, the stent 20511 may be manufactured by laser cutting from a single prefabricated tube with uniform wall thickness, simplifying the manufacturing process of the stent 20511. Furthermore, the uniform thickness of the solid structures at all positions of the stent 20511 also means that the wall thickness of the stent 20511 after being collapsed is uniform, thereby guaranteeing uniform dimensions of the entire pump head after collapsed.

As shown in FIGS. 1 and 2, the edge struts 111 are generally linear. The meshes 14 are formed by multiple edge struts 111, including the first edge struts 15 and the second edge struts 16 extending in different directions. Each of the meshes 14 includes a pair of parallel first edge struts 15 and a pair of parallel second edge struts 16. The first edge struts 15 and the second edge struts 16 have the same length.

The first edge struts 15 and the second edge struts 16 located at the same position in the axial direction X are sequentially connected end to end in the circumferential direction to form a serrated ring 17. The number of the serrated rings 17 is greater than or equal to three, for example, may be three, four as shown in FIG. 1, five, or six as shown in FIG. 12, n, or the like. Preferably, the number of the serrated rings 17 is four. As mentioned above, the maximum circumferential width of the edge strut 111 included in two serrated rings 17 at the ends is smaller than that of the edge strut 111 included in the other serrated rings 17. It may also be understood that the circumferential width of the edge strut 111 included in the two serrated rings 17 at the middle part is greater than that of the edge strut 111 included in the two serrated rings 17 at the both ends. Certainly, the minimum circumferential width of the edge strut 111 included in the two serrated rings 17 at the ends is also smaller than that of the edge strut 111 included in the other serrated rings 17, and further, the minimum circumferential width of the edge strut 111 included in the two serrated rings 17 at the middle part is greater than or equal to the maximum circumferential width of the edge strut 111 included in the two serrated rings 17 at the both ends, so as to make the support rigidity of the body portion 11 uniform.

Further, the edge struts 11 included in at least one serrated ring 17 (the number is greater than or equal to 1) other than the two serrated rings 17 at the ends have the same maximum circumferential width, and the same minimum circumferential width. In short, under the same comparison dimension, the edge struts 11 at the middle part have the same circumferential width, which is greater than the circumferential width of the edge struts 11 at the ends.

This structural design ensures that the edge struts 11 at the middle part have the same structure, eliminating the need to change the manufacturing process at the middle part due to changes in the width of the edge struts 11, thereby appropriately simplifying the manufacturing complexity of the body portion 11. In addition, it may also be ensured that the edge struts 111 at the middle part have a larger circumferential width than the edge struts 111 at the ends under the same comparison dimension, thereby enhancing the support rigidity at the middle part of the body portion 11 and promoting the uniformity of the overall support rigidity of the body portion 11.

As described above, when the stent 20511 is collapsed, the ends of the edge strut 111 are turned relative to each other. The collapse of the stent 20511 is completed when every two circumferentially adjacent edge struts 111 are substantially abutted against each other at their ends. Therefore, it is undesirable for the two circumferentially adjacent edge struts 111 to abut against each other at their ends until the collapse of the stent 20511 is completed. Alternatively, if the two circumferentially adjacent edge struts 111 abut against each other at their ends too early (referred to as self-interference), the resistance to further collapse of the stent 20511 may increase, thereby affecting the dimensions after collapsed, as shown in FIG. 7. The greater the width of the edge strut 111 at the ends, the more severe the aforementioned self-interference phenomenon becomes.

In view of this, in order to prevent self-interference of the edge strut 111 when collapsed as shown in FIG. 7, an angle between the two circumferentially adjacent edge struts 111 (i.e., the first edge strut 15 and the second edge strut 16) of the same serrated ring 17 is reduced adjacent to the connection point.

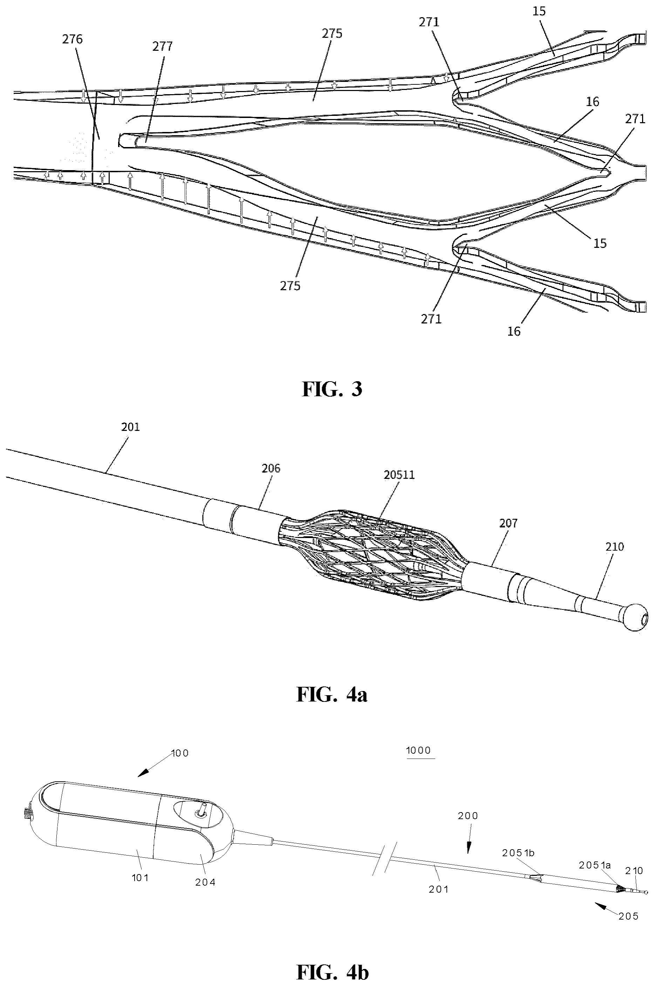

Specifically, as shown in FIG. 3, a first avoidance slot 271 extending in the axial direction X is formed by inner sides at the connection point between the two circumferentially adjacent edge struts 111 (i.e., the first edge strut 15 and the second edge strut 16). The sides of the two edge struts 111 defining the first avoidance slot 271 are substantially parallel

In this case, when the stent 20511 is expanded, the two adjacent edge struts 111 are not directly connected at an angle at the connection point, but are spaced apart internally a distance before being connected at the connection point. Thus, when the stent 20511 is collapsed, this distance provides space for the two adjacent edge struts 111 to turn closer to each other, preventing the portions of the two adjacent edge struts 111 adjacent to the connection point from abutting against each other prematurely. Consequently, the self-interference of the internal structure of the stent 20511 when collapsed is prevented, significantly reducing the resistance to collapse the stent 20511. Furthermore, the collapsed stent 20511 does not exhibit excessive rebound force caused by the aforementioned self-interference, ensuring that the collapsed stent 20511 has a smaller size and better dimensional stability.

The term "substantially" may be understood as "approximately", meaning that the angle between the sides of the two edge struts 111 may fluctuate within a certain range, such as 0 degree to 5 degrees. For example, the sides of the two edge struts 111 forming the first avoidance slot 271 are strictly parallel to each other, i.e., the angle is 0. Alternatively, the angle between the sides of the two edge struts 111 forming the first avoidance slot 271 is smaller than or equal to 5 degree, meaning that they are not strictly parallel

In fact, to avoid the self-interference, providing the first avoidance slot 271 is only one of feasible solutions. Under the guidance of the technical essence of this solution, it may be understood that it is sufficient to space apart the ends of the two adjacent edge struts 11 of the serrated ring 17 in the circumferential direction.

In an embodiment with the first avoidance slot 271 being provided as shown in FIG. 13A, the avoidance slot 271 having a certain depth and a width d is formed by the edge struts 15 and 16 at their ends. In an embodiment illustrated in FIG. 13B, no avoidance slot 17 is formed by the edge struts 15 and 16 at their ends. Instead, the edge struts 15 and 16 are connected by a bar 156 extending generally in the circumferential direction at their ends, and their ends are still separated by a circumferential distance d.

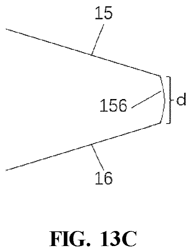

Furthermore, although not shown in FIGS. 13A to 13C, the edge struts 15 and 16 all have smooth transitions at their ends. For example, the connection between the edge struts 15 and 16 and a horizontal portion (not shown), the connection between the horizontal portion and a vertical portion in FIG. 13A, and the connection between the edge struts 15 and 16 and the bar 156 in FIGS. 13B and 13C are all transitioned with chamfered or rounded corners. Furthermore, the bar 156 may also have an arcuate shape, convex away from the ends of the edge struts 15 and 16, as shown in FIG. 13C.

As described above, the edge strut 111 is substantially linear. On this basis, the inner sides of two circumferentially adjacent edge struts 111 at their connection of ends are generally parallel in the following manner. As shown in FIG. 3, the edge strut 111 may include three sections: a middle section and two end sections. The middle section is linear, and the end sections are not aligned with the middle section. Specifically, the end sections have a slightly outward-expanding configuration. As a result, an included angle α1 between the middle sections of the two adjacent edge struts 111 is relatively large (non-zero), and an included angle α2 between the end sections of the two adjacent edge struts 111 is relatively small (α2 < α1), being approximately 0 degree or 0 degree.

As shown in FIGS. 1 and 2, the serrated ring 17 has multiple distal apexes 18 and multiple proximal apexes 19 arranged alternately in the circumferential direction. Multiple axial connecting bars 21 are provided between every two adjacent serrated rings 17 and are arranged in the circumferential direction. Both ends of the axial connecting bar 21 are respectively connected to the distal apex 18 and the proximal apex 19 that are opposite to each other in the axial direction X. The axial connecting bars 21 extend in the axial direction X and connect two adjacent serrated rings 17 in the axial direction X, in which case the previously separate serrated rings 17 are connected to each other, forming the body portion 11 of the stent 20511 while further connecting the inlet portion 12 and the outlet portion 13 to the body portion 11, thereby forming a complete stent 205 11. By matching the structural design with a variable circumferential width of the edge strut 111, the stent 20511 is enabled to have both excellent collapse compliance and excellent rigidity.

The axial connecting bars 21 form a part of the meshes of the body portion 11. Therefore, to maintain uniform rigidity across the body portion 11, the circumferential width of the axial connecting bars 21 is designed to follow the circumferential width of the edge strut 111. That is, the circumferential width of the axial connecting bar 21 close to the middle part is greater than that of the axial connecting bar 21 away from the middle part. Alternatively, the circumferential width of the axial connecting bar 21 increases towards the middle part of the body portion 11, and decrease towards the ends or end positions of the body portion 11.

Likewise, the axial connecting bars 21 at the same position in the axial position X have the same circumferential width, so that the rigidity of the stent 20511 at the same position in the axial direction X is uniform.

Furthermore, since the axial connecting bars 21 do not undergo turning similar to the ends of the edge struts 111 during the collapse and expansion of the stent 20511, the circumferential width of each of the axial connecting bars 21 is uniform and does not vary, unlike the variable circumferential width of the edge struts 111.

Aside from the aforementioned considerations, the uniform width of each of the axial connecting bars 21 also minimizes the impact on the rigidity of the body portion 11 and simplifies the manufacturing process. Specifically, since the axial connecting bars 21 at the same position in the axial direction have the same circumferential width, the uniform width of each of the axial connecting bars 21 ensures that the rigidity of the serrated rings 17 on both axial sides is determined solely by the width of the edge struts 111. This prevents excessive variables from being involved in adjusting the rigidity of the body portion 11, thereby ensuring more accurate rigidity adjustment of the body portion 11.

Furthermore, as mentioned above, since the axial connecting bars 21 at the same position in the axial direction have identical and uniform circumferential width, the manufacturing process for the axial connecting bars 21 at the same position in the axial direction is standardized, thereby simplifying the manufacturing process.

As shown in FIG. 1, the inlet portion 12 includes multiple first struts 272 extending distally from the farthest distal apexes 18. Each of the farthest distal apexes 18 is connected to one first strut 272, and two adjacent first struts 272 converge at an end (distal end) away from the body portion 11 to form a first intersection 273. Similarly, to prevent self-interference between two circumferentially adjacent first struts 272 when collapsed, an included angle between the two first struts 272 forming the first intersection 273 decreases towards the first intersection 273. Specifically, a second avoidance slot 274 extending in the axial direction X is formed by inner sides at the first intersection 273. The sides of the two first struts 272 defining the second avoidance slot 274 are substantially parallel

Similarly, the outlet portion 13 includes multiple second struts 275 extending proximally from the closest proximal apexes 19. Each of the closest proximal apexes 19 is connected to one second strut 275, and two adjacent second struts 275 converge at an end (proximal end) away from the body portion 11 to form a second intersection 276. A third avoidance slot 277 extending in the axial direction X is formed by inner sides at the second intersection 276. The sides of the two second struts 275 defining the third avoidance slot 277 are substantially parallel

The manner in which the first strut 272 and the second strut 275 form the avoidance slot with substantially parallel inner sides at the connection between their ends may be referred to the above description and is not repeated here.

As shown in FIG. 1, a proximal connecting portion 23 is further provided proximal to the outlet portion 13. In the present embodiment, the proximal connecting portion 23 includes multiple proximal connecting legs 231 spaced apart in the circumferential direction. The proximal connecting legs 231 are configured to be connected to the catheter 201 or a proximal bearing housing 206 to securely connect the stent 20511 to the catheter 201.

As in the known embodiment provided in publication No. CN 114588533A, the distal connection structure of the stent utilizing discrete legs (specifically, multiple legs spaced apart in the circumferential direction) constitutes the conventional technology. To maintain a strong, fixed connection with the catheter or the proximal bearing housing, however, the proximal connection structure of the stent currently predominantly utilizes a structure with a circumferentially continuous collar. The reason is that the structure with the circumferentially continuous collar does not tilt up due to leverage when the pump head is collapsed, thereby always maintaining a fixed connection with the catheter. In view of this, the proximal connection structure of the stent cannot utilize the identical or similar discrete legs as the distal connection structure.

In addition, to simplify the process as much as possible, the stent may be fabricated by carving or laser cutting a single prefabricated tube, with a connecting collar being provided at the proximal end of the stent to achieve connection and fixation with the tube or the proximal bearing housing. Consequently, the sleeve-shaped connecting collar has the same diameter as a part of the tube prefabricated to form the stent.

To ultimately achieve a compact size for easy placement and intervention, the prefabricated tube is relatively small in diameter. Further, meeting the requirement for a larger final expanded diameter of the stent results in increased engraving, leading to narrower width and reduced rigidity of the edge struts of the stent. In contrast, meeting the requirements for the width and rigidity of the edge struts of the stent results in less engraving to remove, leading to a smaller expanded diameter.

Additionally, a prefabricated tube with larger diameter may be carved to form the stent, with the proximal connecting collar being subsequently thinned down. However, this approach results in waste generation, high costs, and complex process.

Instead of using the connecting collars in the conventional technology which are at least partially continuous in the circumferential direction, by providing multiple proximal connecting legs 231 spaced apart in the circumferential direction, that is, distributing the multiple proximal connecting legs 231 in a discrete manner, the above-mentioned issue can be effectively solved. The details are as follows.

As mentioned above, if a circumferentially continuous connecting collar is adopted (in which case the connecting collar constitutes the proximal portion of the prefabricated tube), the diameter of the connecting collar determines the diameter of the collapsed stent. In other words, the diameter of the collapsed stent 20511 is constrained by the diameter of the proximal connecting collar, that is, by the diameter of the prefabricated tube. If the diameter of the prefabricated tube is large, it is difficult to achieve the small diameter of the collapsed stent 20511, thereby failing to meet the requirement for small intervention size of the pump head. If the diameter of the prefabricated tube is small, although it is possible to achieve the small collapse size and intervention size of the stent 20511, it cannot achieve the large expansion diameter and support rigidity during expansion of the stent 205 11. The reason is as follows. To achieve the large expansion diameter, a significant amount of material needs to be removed from the tube through cutting, resulting in a smaller width of the bars of the stent 20511, particularly the body portion 11 of the stent 20511, which leads to a decrease in support rigidity after expanded. In contrast, to achieve the large support rigidity after expanded, a larger width of the bars of the stent 205 11, especially the body portion 11 of the stent 20511, is required, which means that the amount of the material removed from the tube during cutting cannot be too much, and in turn results in an insufficient expansion diameter of the stent 20511.

In contrast, the stent 20511 according to the present embodiment no longer utilizes a structure with a circumferentially continuous collar at its proximal end, but instead utilizes a structure with multiple discrete legs that are not interconnected. Consequently, the diameter of the collapsed stent 20511 is not constrained by the diameter of the prefabricated tube, meaning that the stent 20511 may be manufactured by laser cutting a prefabricated tube with a relatively large diameter. Since the diameter of the selected prefabricated tube is larger than that used in the conventional technology, the amount of the material removed through cutting is reduced to achieve the same expanded diameter, increasing the width of the bar of the body portion 11 of the stent 20511 and thereby improving the support rigidity of the stent 20511. Alternatively, to achieve the same support rigidity, the amount of the material being removed through cutting may be increased, reducing the width of the bar of the body portion 11 of the stent 20511 and thereby increasing the expanded diameter of the stent 20511.

It should be noted that the stent 20511 according to the present embodiment is fabricated as a whole by laser cutting a prefabricated tube. That is, compared with the conventional technology, which only uses laser cutting to form the body portion 11 and the distal connecting legs of the stent 20511, the present embodiment utilizes laser cutting to form the entire structure of the stent 20511, including the body portion 11, the proximal connecting legs 231, and the distal connecting legs 221. This simplifies the manufacturing process of the stent 20511.

The stent 20511 formed by using the above method has a hollow cylindrical structure (at this stage, no distinction is made among the body portion, the proximal connecting legs 231, and the distal connecting legs 221), with equal outer diameter of all portions in the axial direction. Subsequently, the hollow cylindrical structure (referred to as the pre-molded stent) undergoes a shaping process to obtain the final stent structure. Specifically, the pre-molded stent is placed over an internal shaping mold, which is then sleeved with an external shaping mold (the external contour shape of the internal shaping mold and the shape of the internal cavity of the external shaping mold may refer to the shape of the stent shown in FIGS. 1 or 2). Subsequently, a heat treatment process is applied to the pre-molded stent, heating it to a phase transition temperature of the stent material (e.g., nickel-titanium alloy) and maintaining the temperature for a period of time, followed by cooling and demolding to obtain the final stent.

Therefore, compared with the conventional technology, the present embodiment employs a structure with the proximal discrete connecting legs. This allows the body portion 11 of the stent 20511 to maintain a compact size when radially collapsed, while also allows the body portion 11 of the stent 20511 to have a larger expanded diameter and greater support rigidity when radially expanded. Moreover, compared with the stent with the proximal connecting collar carved from the larger-diameter prefabricated tube, the present embodiment generates no waste material, reduces costs, and simplifies the process.

The proximal connecting leg 231 includes a proximal bar body 232 connected to the outlet portion 13 and a first extension 236 connected to the proximal bar body 232. The proximal bar body 232 extends in the axial direction X, and the first extension 236 extends in the circumferential direction, such that the first extension 236 is perpendicular to the proximal bar body 232. The circumferential width of the first extension 236 is greater than the circumferential width of at least part of the proximal bar body 232, thereby forming a generally T-shaped structure of the proximal connecting leg 231 to match with a groove on an outer wall of the proximal bearing housing 206, thereby achieving a fixed connection between the stent 20511 and the catheter 201.

The proximal bar body 232 includes a first bar portion 233 connected to the outlet portion 13, and a second bar portion 234 with its opposite ends connected to the first bar portion 233 and the first extension 236, respectively. The circumferential width of the second bar portion 234 is smaller than that of the first bar portion 233 and the first extension 236. Consequently, a narrowing portion is formed at the second bar portion 234, which engages with the groove on the outer wall of the proximal bearing housing 206 to secure the proximal connecting leg 231 to the proximal bearing housing 206. The entire structure of the second bar portion 234 in the axial direction is fixedly connected to the catheter 201 or the proximal bearing housing 206. A portion (the proximal portion) of the first bar portion 233 is fixedly connected to the catheter 201 or the proximal bearing housing 206, and the other portion (the distal portion) extends distally outside the catheter 201 or the proximal bearing housing 206.

Therefore, in the proximal connecting leg 231, the proximal portion of the first bar portion 233 with a greater width is fixedly connected to the catheter 201 or the proximal bearing housing 206, providing support for the cantilevered distal portion of the first bar portion 233 extending outside the catheter 201 or the proximal bearing housing 206, so that the proximal cantilever structure of the stent 20511 is proved with improved support rigidity (avoiding support for the cantilever structure by the second bar portion 234 with a smaller width), thereby enhancing the support rigidity of the proximal cantilever structure of the stent 20511. In this way, the body portion 11 of the stent 20511 is supported by the cantilever portion with higher support strength, thereby ensuring that the body portion 11 of the stent 20511 has higher support rigidity in the expanded state.

A proximal transition portion 235 is provided between the first bar portion 233 and the second bar portion 234. The circumferential width of the proximal transition portion 235 gradually increases from being equal to the circumferential width of the second bar portion 234 to being equal to the circumferential width of the first bar portion 233 in a direction from the proximal end to the distal end, thereby enhancing the rigidity of the second bar portion 234 with a smaller circumferential width.

Similarly, a distal connecting portion 22 is further provided at the distal end of the inlet portion 12. The distal connecting portion 22 includes multiple distal connecting legs 221 spaced apart in the circumferential direction. The distal connecting leg 221 includes a distal bar body 222 connected to the inlet portion 12 and a second extension 226 connected to the distal bar body 222. The distal bar body 222 extends in the axial direction X, and the second extension 226 extends in the circumferential direction, such that the second extension 226 is perpendicular to the distal bar body 222.

The circumferential width of the second extension 226 is greater than the circumferential width of at least part of the distal bar body 222, thereby forming a generally T-shaped structure of the distal connecting leg 221 to match with a groove on an outer wall of the distal bearing housing 207, thereby achieving a fixed connection between the stent 20511 and the distal bearing housing 207.

The distal bar body 222 includes a third bar portion 223 connected to the inlet portion 12, and a fourth bar portion 224 with its opposite ends connected to the third bar portion 223 and the second extension 226, respectively. The circumferential width of the fourth bar portion 224 is smaller than that of the third bar portion 223 and the second extension 226. Consequently, a narrowing portion is formed at the fourth bar portion 224, which engages with a groove on the outer wall of the distal bearing housing 207 to secure the distal connecting leg 221 to the distal bearing housing 207.

A distal transition portion 225 is provided between the third bar portion 223 and the fourth bar portion 224. The circumferential width of the distal transition portion 225 gradually decreases from being equal to the circumferential width of the third bar portion 223 to being equal to the circumferential width of the fourth bar portion 224 in a direction from the proximal end to the distal end, thereby enhancing the rigidity of the fourth bar portion 224 with a smaller circumferential width.

The distal connecting leg 221 and the proximal connecting leg 231 adopt the identical or similar structural design and can achieve basically the same technical effect. Specific details may be referred to the above description and are not repeated here.

The catheter pump 1000 according to the embodiments of the present application is configured to perform partial blood pumping of the heart. In scenarios where left ventricular assist is applicable, the catheter pump 1000 pumps blood from the left ventricle into the aorta, providing support for blood circulation and reducing the cardiac workload on the subject, or providing additional continuous pumping power support when the blood-pumping capacity of the heart is insufficient. Certainly, the catheter pump 1000 may also be introduced into other target locations in the subject as desired during interventional surgery, such as the right ventricle, blood vessels, or other organs.

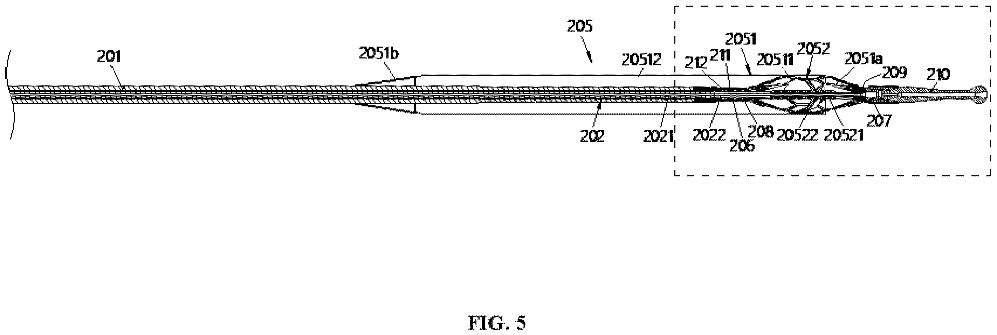

As shown in FIGS. 4a and 4b, the catheter pump 1000 includes a power assembly 100 and a working assembly 200. The power assembly 100 includes a housing 101, a motor (not shown) housed in the housing 101, and an driving element (not shown) driven by the motor. As shown in FIG. 5, the working assembly 200 includes a catheter 201, a driving shaft 202 extending through the catheter 201, a driven element connected to a proximal end of the driving shaft 202, and a driving catheter handle 204 and a pump head 205 connected to the proximal and distal ends of the catheter 201, respectively. The pump head 205 may be delivered through the catheter 201 to a desired location in the heart, such as the left ventricle, to pump blood. The pump head 205 includes a pump housing 2051 having at least one blood inlet 2051a and at least one blood outlet 2051b, and an impeller 2052 housed in the pump housing 2051. The blood inlet 2051a is located at the distal end of the pump housing 2051, and the blood outlet 2051b is located at the proximal end of the pump housing 2051. The motor is located at the proximal end of the catheter 201 and drives the impeller 2052 to rotate and pump blood via the driving shaft 202. The impeller 2052 is connected to the distal end of the driving shaft 202. The impeller 2052 rotates to draw blood through the blood inlet 2051a into the pump housing 2051 and discharge blood from the pump housing 2051 through the blood outlet 2051b.

The pump housing 2051 is connected to the distal end of the catheter 201, and the impeller 2052 is connected to the distal end of the driving shaft 202. The pump housing 2051 includes a film 20512 that defines a blood flow channel and a collapsible stent 20511 that supports the expansion of the film 20512. The proximal end of the stent 20511 is connected to the distal end of the catheter 201. The stent 20511 is the stent 20511 described in any of the above embodiments, and the proximal connecting portion 23 of the stent 20511 is connected to the distal end of the catheter 201.

The film 20512 is elastic and covers a portion of the stent 205 11. The impeller 2052 is housed in the stent 20511 and located inside the film 20512. The stent 20511 is supported at the distal end of the film 20512, with a portion of the stent 20511 located outside the distal end of the film 20512 and the other portion of the stent 20511 located inside the film 20512. The impeller 2052 is mostly located in the body portion 11 of the stent 20511, with both ends (primarily hubs 20521) extending into the inlet portion 12 and the outlet portion 13.

The film 20512 may cover the middle and rear portions of the stent 20511. The meshes 14 in a front portion of the stent 20511 not covered by the film 20512 form the blood inlet 2051a. A rear end of the film 20512 covers the distal end of the catheter 201. The blood outlet 2051b is at least one opening formed at the rear end of the film 20512.

The film 20512 includes a cylindrical segment as its main structure and a tapered segment located proximally to the cylindrical segment. The proximal end of the tapered segment is positioned outside the catheter 201 and fixed to the outer wall of the catheter 201. The catheter 201, by virtue of the proximal bearing housing 206 at its distal end, is connected to the proximal end of the stent 20511. The proximal bearing housing 206 houses a proximal bearing 208 that rotatably supports the driving shaft 202.

The impeller 2052 includes a hub 20521 and blades 20522 supported on an outer wall of the hub 20521. The blades 20522 are made of flexible material and, together with the stent 20511 made of nickel and titanium memory alloy and the film 20512, form a collapsible pump head 205.

The distal bearing housing 207 is provided at the distal end of the stent 20511. A distal bearing 209 is disposed in the distal bearing housing 207 to provide rotational support for the distal end of the driving shaft 202. The driving shaft 202 includes a flexible shaft 2021 that extends through the catheter 201, and a rigid shaft 2022 that extends through a hollow passage of the hub 20521 and connects to the distal end of flexible shaft 2021. The hub 20521 of the impeller 2052 is sleeved onto the rigid shaft 2022, with the proximal and distal ends of the rigid shaft 2022 respectively passing through the proximal bearing 208 and the distal bearing 209. Thus, the rigid shaft 2022 is supported at both ends by the two bearings. Combined with its high rigidity, the rigid shaft 2022 provides rigid support for the impeller 2052 in the pump housing 2051, enabling the impeller 2052 to be optimally maintained in the pump housing 2051, and thereby guaranteeing the stability of the impeller 2052 in the pump housing 2051.

A stopper 211 is provided on the rigid shaft 2022 proximal to the proximal bearing 208 to limit the distal movement of the rigid shaft 2022 and the impeller 2052, thereby preventing the impeller 2052 from moving distally due to a counterforce from the blood during its rotational pumping. A position-limiter 212 is also provided on the rigid shaft 2022 proximal to the stopper 211 to limit the proximal movement of the rigid shaft 2022 and the stopper 211, thereby preventing the stopper 211 from abrading the distal end of the catheter 201 and causing the release of particulate matter.

A non-invasive support member 210 made of flexible material is provided at the distal end of the distal bearing housing 207. The non-invasive support member 210 is supported on the inner wall of the ventricle in a non-invasive or atraumatic manner, separating the blood inlet 2051a of the pump head 205 from the inner wall of the ventricle, preventing the blood inlet 2051a of the pump head 205 from adhering to the inner wall of the ventricle due to a reaction force of the blood during the operation of the pump head 205, thereby ensuring an effective pumping area.

The driving catheter handle 204 is detachably connected to the power assembly 100, by adopting a locking nut or through a snap-fit connection as provided in US9421311B2. The driven member is coupled to the driving member in a non-contact manner to transmit the rotational power of the motor to the driving shaft 202, thereby driving the impeller 2052 to rotate and pump blood. As mentioned above, the driven member may be coupled to the driving member in a magnetic coupling manner as provided in CN103120810B or CN101820933B, or by virtue of an Eddy Current Coupling as provided in CN216061675U or CN114452527A, which is not limited in the present embodiment.

The catheter pump 1000 described above employs an external motor. Based on the foregoing, the catheter pump 1000 may also employ a configuration with an internal motor. In this case, the motor is connected to the distal end of the catheter 201, without an elongate, flexible driving shaft 202 passing through the catheter 201. Instead, the motor drives the impeller 2052 via a rigid short shaft, magnetic coupling, or other means.

The above examples, which are described specifically and in detail, only illustrate several implementations of the present application, and should not be construed as limiting the protection scope of the present application. It should be noted that various changes and improvements made by those of ordinary skill in the art without departing from the concept of the present application also fall within the protection scope of the present application. Therefore, the protection scope of the present application shall be subjected to the claims.

## Claims

1. A catheter pump, comprising:
a catheter; and
a pump head configured to be delivered through the catheter to a desired location in a heart for pumping blood, and comprising: a pump housing having at least one blood inlet and at least one blood outlet; and an impeller housed in the pump housing and rotatable to draw blood through the blood inlet into the pump housing and to discharge blood through the blood outlet;
wherein the pump housing comprises a stent operable between a radially collapsed state and a radially expanded state, and the stent in the radially expanded state comprises: a body portion having a substantially cylindrical shape; an inlet portion located at a distal end of the body portion in an axial direction; and an outlet portion located at a proximal end of the body portion in the axial direction,
wherein the inlet portion and the outlet portion are connected to and support the body portion; and
wherein the body portion is provided with a plurality of meshes, each of which is defined by at least two pairs of oppositely-arranged edge struts, all of the edge struts have a same thickness in a radial direction, and each of the edge struts has a circumferential width greater at both ends than at a middle portion in an axial direction of the edge strut.

2. The catheter pump according to claim 1, wherein the circumferential width of the edge strut decreases from the both ends to the middle portion in a curved manner.

3. The catheter pump according to claim 2, wherein the edge strut has two circumferentially opposite sides that gradually narrow from the both ends to the middle portion.

4. The catheter pump according to claim 3, wherein the two circumferentially opposite sides of the edge strut narrow from the both ends to the middle portion at a same tendency and to a same extent.

5. The catheter pump according to claim 4, wherein the edge strut has the narrowest circumferential width approximately at its middle portion.

6. The catheter pump according to claim 2, wherein the circumferential width of the edge strut close to a middle part of the body portion is greater than that of the edge strut away from the middle part of the body portion.

7. The catheter pump according to claim 6, wherein the edge struts located at a same position in an axial direction of the body portion have a same circumferential width.

8. The catheter pump according to claim 1, wherein a minimum circumferential width of the edge strut close to a middle part of the body portion is greater than or equal to a maximum circumferential width of the edge strut away from the middle part of the body portion.

9. The catheter pump according to claim 1, wherein a plurality of edge struts located at a same position in the axial direction of the body portion are connected end to end in a circumferential direction to form a serrated ring, and a plurality of serrated rings are arranged and connected together in the axial direction; and
wherein the serrated ring has a plurality of distal apexes and a plurality of proximal apexes arranged alternately in the circumferential direction, a plurality of axial connecting bars are provided between every two adjacent serrated rings and are arranged in the circumferential direction, opposite ends of the axial connecting bar are respectively connected to the distal apex and the proximal apex opposite to each other in the axial direction, and a circumferential width of the axial connecting bar close to a middle part of the body portion is greater than that of the axial connecting bar away from the middle part of the body portion.

10. The catheter pump according to claim 9, wherein on a same serrated ring, ends of every two adjacent edge struts are spaced apart in the circumferential direction; and
wherein preferably, a first avoidance slot is formed at ends of the every two adjacent edge struts between which an angle is formed, and extends in the axial direction, and has opposite sides that are substantially parallel over a portion of its length.
